(19) Europäisches Patentamt
European Patent Office
Office européen des brevets

(11) Publication number: **0 234 499 A2**

# (12) EUROPEAN PATENT APPLICATION

(21) Application number: 87102316.4

(22) Date of filing: 18.02.87

(51) Int.Cl.³: C 07 D 249/08
C 07 D 233/60, C 07 D 231/12
C 07 D 403/06, A 61 K 31/41
A 61 K 31/415
//C07D405/06

(30) Priority: 28.02.86 JP 45011/86

(43) Date of publication of application:
02.09.87 Bulletin 87/36

(84) Designated Contracting States:
AT BE CH DE ES FR GB GR IT LI LU NL SE

(71) Applicant: SHIONOGI SEIYAKU KABUSHIKI KAISHA
12, Dosho-machi, 3-chome Higashi-ku, Osaka-shi
Osaka 541(JP)

(72) Inventor: Ogata, Masaru
8-20-8, Sumiyoshiyamate Higashinada-ku
Kobe-shi Hyogo(JP)

(72) Inventor: Matsumoto, Hiroshi
5-10-25, Yamatedai
Ibaraki-shi Osaka(JP)

(72) Inventor: Kida, Shiro
9-12-712, 3-chome Higashiikuni Yodogawa-ku
Osaka-shi Osaka(JP)

(72) Inventor: Tawara, Katsuya
1-1-814, Higashiota
Ibaraki-shi Osaka(JP)

(72) Inventor: Shimizu, Sumio
7-20-904, 5-chome, Uozakikitamachi
Higashinada-ku Kobe-shi Hyogo(JP)

(74) Representative: Vossius & Partner
Siebertstrasse 4 P.O. Box 86 07 67
D-8000 München 86(DE)

## (54) Antifungal azolylpropanol derivatives.

(57) A compound of the formula:

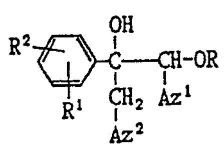

(I)

(wherein $Az^1$ and $Az^2$ each is imidazolyl, triazolyl or pyrazolyl, each optionally substituted by methyl or halogen;
R is $C_1$–$C_5$ alkyl or $C_1$–$C_5$ haloalkyl;
$R^1$ is hydrogen, halogen or trifluoromethyl and
$R^2$ is hydrogen or halogen)
or its salt, being useful as as antimycotic agent, is provided.

EP 0 234 499 A2

Croydon Printing Company Ltd.

Case: G6042-EP/HW
Shionogi Seiyaku K.K.,
J A P A N

PATENTANWÄLTE
SIEBERTSTR. 4
8000 MÜNCHEN 86

0234499
18th February 1987

# ANTIFUNGAL AZOLYLPROPANOL DERIVATIVES

The present invention relates to antifungal azolylpropanol derivatives. More particularly, this invention is directed to azolylpropanol derivatives which have been found to be particularly effective in the treatment of human or veterinary mycosis, to their preparation, to their use and to pharmaceutical formulations containing the compounds.

Azolylpropanol derivatives are known from the Japanese Unexamined Patent Publications Nos. 54-130588, 58-32868, 59-176266 and 60-139676.

Since some compounds, however, may induce strong adverse actions such as hepatotoxicity or teratogenesis depending upon the structure, they are not always satisfactory.

According to the present invention there are provided azolylpropanol derivatives of the general formula I:

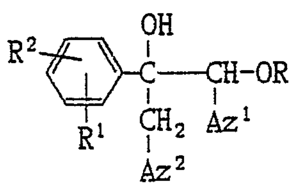

( I )

(wherein $Az^1$ and $Az^2$ each is imidazolyl, triazolyl or pyrazolyl, each optionally substituted by methyl or halogen;

$R$ is $C_1$-$C_5$ alkyl or $C_1$-$C_5$ haloalkyl;

$R^1$ is hydrogen, halogen or trifluoromethyl and

$R^2$ is hydrogen or halogen)

and their salts.

The compounds of the present invention have an excellent antimycotic activity, and they are available for human or veterinary medicines. Accordingly the invention also provides an antimycotic composition comprising as an active ingredient 0.1 to 95 % by weight of at least one compound of the formula (I) associated with one or more pharmaceutically acceptable carriers, diluents and / or excipients.

This invention also comprises the compounds of the general formula (I) for use in the treatment of mycosis.

This invention further provides a process for preparing a compound of the formula ( I ) which comprises reacting a compound of the general formula II:

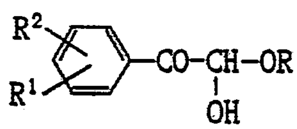

( II )

(wherein R, $R^1$ and $R^2$ each has the same meaning as defined above)
with an azole introducing agent to give a compound of the general formula III:

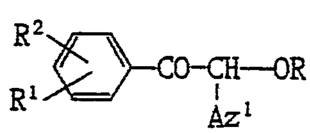

( III )

(wherein R, $R^1$, $R^2$ and $Az^1$ each has the same meaning as defined above),
subjecting said compound ( III ) to an oxirane introducing reaction

to give a compound of the general formula IV:

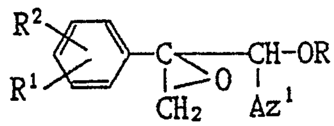 (IV)

(wherein R, $R^1$, $R^2$ and $Az^1$ each has the same meaning as defined above),
and reacting said compound (IV) with an azole in a solvent.

The terms used in the above definition will be illustratively explained below.

The term "$C_1$-$C_5$ alkyl" herein employed refers to a straight or branched saturated aliphatic hydrocarbon radical such as methyl, ethyl, n-propyl, isobutyl, sec-butyl, tert-butyl, n-pentyl, isopentyl, sec-pentyl, neo-pentyl or tert-pentyl.

The term "$C_1$-$C_5$ haloalkyl" herein employed refers to a said $C_1$-$C_5$ alkyl substituted by halogen such as trifluoromethyl, difluoromethyl, trifluoroethyl, monochloropropyl and trichlorobutyl.

The term "halogen" includes fluorine, chlorine, bromine and iodine. The substituent on the azole ring includes methyl and halogen.

The term "azole" means imidazolyl, triazolyl or pyrazolyl, each optionally substituted by methyl or halogen.

In addition, the process for preparing the compound (I) is shown by the scheme as follows.

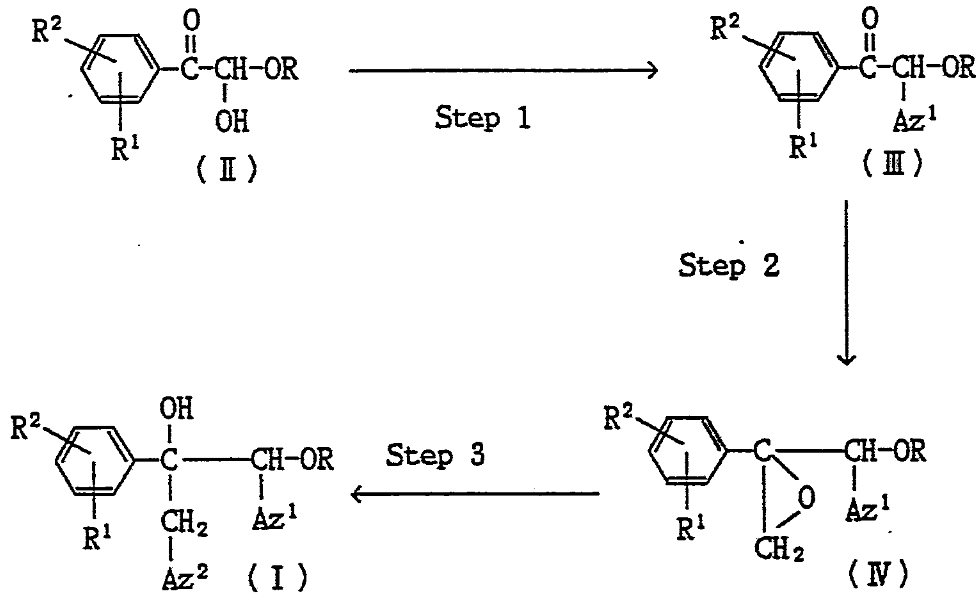


(wherein R, $R^1$, $R^2$, $Az^1$ and $Az^2$ each has the same meaning as defined above).

Step 1

Phenylglyoxal hemiacetal ( II ) as a starting material is allowed to react with an azole introducing agent such as N,N'-sulfinylbisazole or N,N'-carbonylbisazole in a solvent such as methylene chloride or acetonitrile at 0 - 50 °C, preferably at room temperature, whereby the azolylmethyl ketone ( III ) can be obtained.

Further, the starting material ( II ) used in this invention can be synthesized according to the method described in the following reference. The reaction of phenylglyoxal with an

alcohol is disclosed in J. Am. Chem. Soc., 79, 6562 (1957).

Step 2

Azolylmethyl ketones ( III ) obtained above are subjected to the formation of oxirane to lead to the oxirane ( IV ). The formation of the oxirane may be carried out using dimethylsulfonium methylide*[1] in dimethyl sulfoxide in the presence of sodium hydride or using dimethyloxosulfonium methylide*[2] in a solution of toluene - 20 % sodium hydroxide in the presence of benzyltriethylammonium chloride.

*[1] Corey et al, J. Am. Chem. Soc., 87, 1353 (1965)

*[2] Hiyama et al, J. Am. Chem. Soc., 97, 1626 (1975)

Step 3

The oxirane ( IV ) obtained above is allowed to react with an azole such as 1H-1,2,4-triazole, 1H-imidazole or 1H-pyrazole, whereby the objective compound ( I ) is obtained. This reaction is performed in a conventional manner, for example, by heating the oxirane ( IV ) with an alkali salt of the azole such as sodium 1H-azole or potassium 1H-azole at 20 - 100 °C in an organic solvent like dimethylformamide. The objective compound ( I ) thus obtained has two asymmetric carbons. In this case, it can exist in the form of two diasteromers, A and B (as a tentative name).

The objective compounds ( I ) can be converted into their pharmaceutically acceptable acid addition salts.

Illustrative acids which can form such salts are organic acids such as citric acid, malic acid, succinic acid, oxalic acid, methanesulfonic acid or the like and inorganic acids such as hydrohalogenic acid, nitric acid, sulfuric acid, phosphoric acid or the like.

The objective compounds ( I ) and their salts show an excellent antimycotic activity and they are valuable as an antimycotic agent in peroral or parenteral route. Further, they can be useful as a fungicide in agriculture or horticulture and also as a veterinary antimycotic agent for treating infection of mycosis in animals.

For clinical use, Compounds (I) or their salts can be administered alone. Generally, however, they are administered in the form of a pharmaceutical formulation in association with one or more pharmaceutically acceptable carriers, diluents and / or excipient selected, depending upon the route of administration and standard pharmaceutical means. For example, they can be orally administered as tablets containing diluents such as starch or lactose, as capsules or as suspensions and can also be parenterally administered in injections such as intravenous injection, muscular injection and subcutaneous injection. Therefor, it is preferable to use a sterile solution containing one or more supplementary substances (e.g. glucose or sodium chloride sufficient for keeping the injection isotonic with the blood). Appropriate daily dosages of the Compound (I) or their salts for human adults may be 5 to 500 mg in oral route.

The abbreviations used in the Examples, Referential Examples and Tables represent the following meanings.

Me : methyl ; Et : ethyl

Example 1

2'4'-Difluoro-2-methoxy-2-(1H-1,2,4-triazol-1-yl)-acetophenone

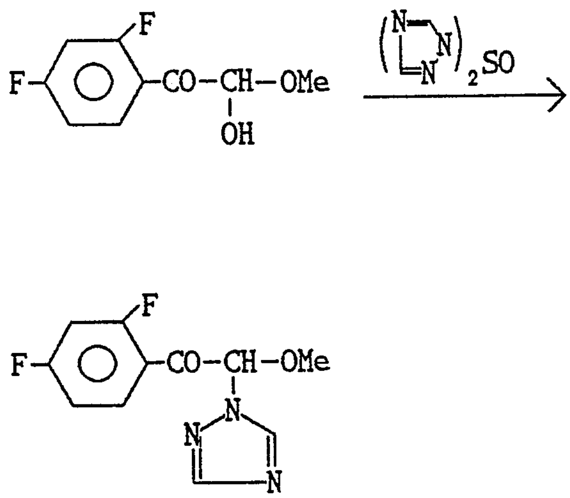

To a solution of 7.8 g of 1H-1,2,4-triazole in 78 ml of dry dichloromethane is added 3.4 g of thionyl chloride. After stirring for 5 min., 4.7 g of 2,4-difluorophenylglyoxal hemiacetal is added thereto and the mixture is stirred at room temperature for 16 hours. The reaction mixture is put into ice water, made alkaline with aqueous sodium hydrogencarbonate and then extracted with methylene chloride. After the organic layer is washed with water, it is dried over anhydrous sodium sulfate and the organic solvent is distilled off. The residue is subjected to silica gel column chromatography, eluting with 2 % methanol - methylene chloride, and the eluate is concentrated. The residue is recrystallized from ethyl acetate and isopropyl ether to give 2',4'-difluoro-2-methoxy-2-(1H-1,2,4-triazol-1-yl)acetophenone (1.8 g

30 %) melting at 98 - 99 °C as crystals.

Anal Calcd. (%) for $C_{11}H_9N_3O_2F_2$ :

C, 52.18; H, 3.58; N, 16.60; F, 15.01

Found (%) : C, 51.98; H, 3.63; N, 16.40; F, 14.92

Examples 2 to 20

The reaction is carried out as in Example 1 to give the azolylmethyl ketones (III).

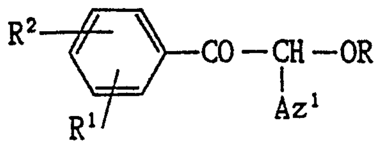
( III )

| Ex. No. | R | $R_1$ | $R_2$ | $Az^1$ *1 | m.p. (°C)/IR (film)($cm^{-1}$) |
|---|---|---|---|---|---|
| 2 | Me | H | H | Tri | 92-93 |
| 3 | Me | H | 4-Cl | Im | 123-125 *2 |
| 4 | Et | H | 4-Cl | Tri | 92-94 *3 |
| 5 | Me | H | 4-Cl | Tri | 127-129 |
| 6 | Me | H | H | Im | 57-60 |
| 7 | Me | 2-F | 4-F | Py | 58-60 |
| 8 | Me | 2-F | 4-F | Im | 168 *2 |
| 9 | Me | H | 4-F | Tri | 81-82 |
| 10 | Me | 2-Cl | 4-Cl | Tri | 89-90 |
| 11 | $CH_2CF_3$ | H | 4-Cl | Tri | 143-144 |
| 12 | Me | H | 4-Cl | Py | 92-93 |
| 13 | Me | H | 2-Cl | Tri | 78-80 |
| 14 | $CH_2-CF_3$ | 2-F | 4-F | Tri | 106-108 |
| 15 | Me | H | 2F | Tri | 91-93 |
| 16 | $CH_2CF_3$ | H | 4-F | Tri | 108-110 |
| 17 | Me | 2-Cl | 4-Cl | Im | oily substance 1720 $cm^{-1}$ |
| 18 | Et | H | 4-F | Tri | 68-69 |
| 19 | Me | 2-F | 4-F | Tri | 94.5-96.5 |
| 20 | Me | H | $4-CF_3$ | Tri | oily substance 1715 $cm^{-1}$ |

*1 The term "Tri" represents 1H-1,2,4-triazol-1-yl, "Im" represents 1H-imidazol-1-yl, and "Py" represents 1H-pyrazol-1-yl, respectively.

*2 Oxalate

*3 GB Pat. Application No. 2,102,796

Example 21

2-(2,4-Difluorophenyl)-1-methoxy-1,3-bis(1H-1,2,4-triazol-1-yl)-2-propanol

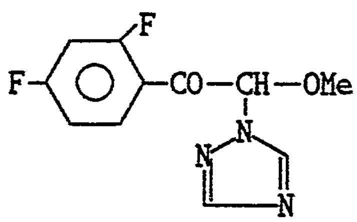

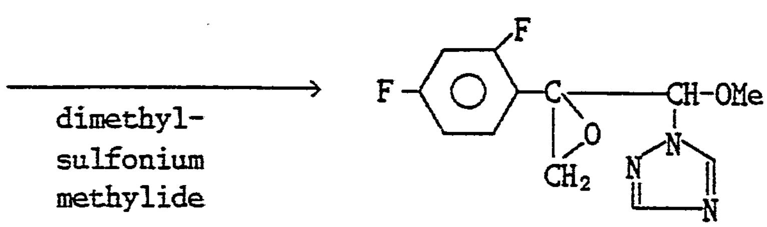

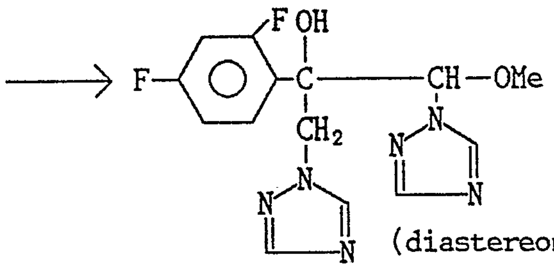

(diastereomer A)

50 % Sodium hydride (mineral oil suspension : 310 mg) is added to 6 ml of dry dimethyl sulfoxide and stirred at 75 °C for 10 min. The mixture is cooled to a temperature of - 10 °C and mixed with a solution of trimethylsulfonium iodide (1.29 g) in 6 ml of dry dimethyl sulfoxide. After 3 min., a solution of 2',4'-difluoro-2-methoxy-2-(1H-1,2,4-triazol-1-yl)acetophenone (800 mg) obtained in Example 1 in 4 ml of dimethyl sulfoxide is added to the mixture, which is stirred at 40 °C for 30 min. The reaction mixture is put into ice water and extracted with ether. The ether layer is washed with water, dried over sodium sulfate and concentrated to give 1-(2,4-difluorophenyl)-2-[1-methoxy-2-(1H-1,2,4-triazol-1-yl)methyl]oxirane.

To a suspension of sodium 1H-1,2,4-triazole made from 1H-1,2,4-triazole (440 mg), 50 % sodium hydride (mineral oil suspension : 310 mg) and dry dimethylformamide (6 ml) is added the oxirane described above and the mixture is stirred at 50 °C for 30 min. Ice water is added to the reaction mixture, which is extracted with methylene chloride. The extract is washed with water, dried over sodium sulfate and concentrated. The residue is subjected to silica gel column chromatography, eluting with 5 % methanol - ethyl acetate and concentrated. The crude crystals

obtained are recrystallized from ethyl acetate - diisopropyl ether to give 106 mg of 2-(2,4-difluorophenyl)-1-methoxy-1,3-bis(1H-1,2,4-triazol-1-yl)-2-propanol (diastereomer A) melting at 160 - 161 °C as crystals.

Yield : 10 %

Anal Calcd. (%) for $C_{14}H_{14}N_6O_2F_2$ :

C, 50.00; H, 4.20; N, 24.99; F, 11.30

Found (%) C, 50.25; H, 4.17; N, 24.70; F, 11.03

Examples 22 to 46

Diastereomer A, one of the two diastereomers of the objective compounds ( I ), is mainly obtained by the same reaction as in Example 21.

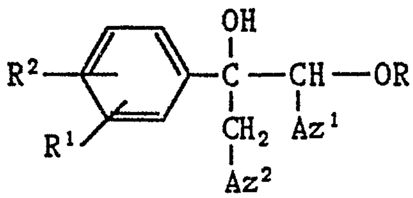

( I )

| Ex. No. | R | R¹ | R² | Az¹ *1 | Az² *1 | m.p. *2 (°C) |
|---|---|---|---|---|---|---|
| 22 | Me | H | H | Im | Tri | 196-198 |
| 23 | Me | H | 4-Cl | Im | Tri | 166-167 |
| 24 | Me | H | 4-F | Tri | Tri | 140-141 |
| 25 | Me | 2-Cl | 4-Cl | Tri | Tri | 151-152 |
| 26 | Me | H | 4-Cl | Tri | Tri | 156-157 |
| 27 | Me | H | 4-F | Py | Tri | 151-152 |
| 28 | Me | H | 4-F | Py | Im | 148-149 |
| 29 | Me | 2-F | 4-F | Py | Im | 148-149 |
| 30 | Me | 2-F | 4-F | Py | Im | 164-165 |
| 31 | Me | H | 4-F | Py | Im | 171-172 |
| 32 | $CH_2CF_3$ | H | 4-F | Tri | Tri | 144-145 |
| 33 | Et | H | 4-F | Tri | Tri | 150-151 |
| 34 | Me | H | 4-Cl | Py | Tri | 144-145 |
| 35 | Me | H | 4-Cl | Py | Im | 131-133 |
| 36 | Me | H | 4-Cl | Py | Im | 192-193 |
| 37 | Me | H | 4-Cl | Py | Tri | 107-110 |
| 38 | Me | H | 4-Cl | Py | Py | 106-107 |
| 39 | Me | H | 4-Cl | Tri | Im | 205-206 |
| 40 | Me | 2-F | 4-F | Tri | Im | 191-192 |
| 41 | Me | H | 4-Cl | Tri | Py | 149-150 |
| 42 | Me | H | 4-F | Tri | Im | 159-160 |
| 43 | Me | 2-F | 4-F | Tri | Tri | 142-146 |
| 44 | Me | 2-Cl | 4-Cl | Tri | Im | 194-195 |
| 45 | Me | H | 4-Cl | 2-Me-Im | Tri | 194-195 |
| 46 | Me | H | 4-Cl | 4-Me-Im | Tri | 198-199 |

*1 The term "Tri" represents 1H-1,2,4-triazol-1-yl, "Im" represents 1H-imidazol-1-yl, and "Py" represents 1H-pyrazol-1-yl, respectively.

*2 Pure diastereomer A

Example 47

2-(2,4-Difluorophenyl)-1-methoxy-1,3-bis(1H-1,2,4-triazol-1-yl)-2-propanol

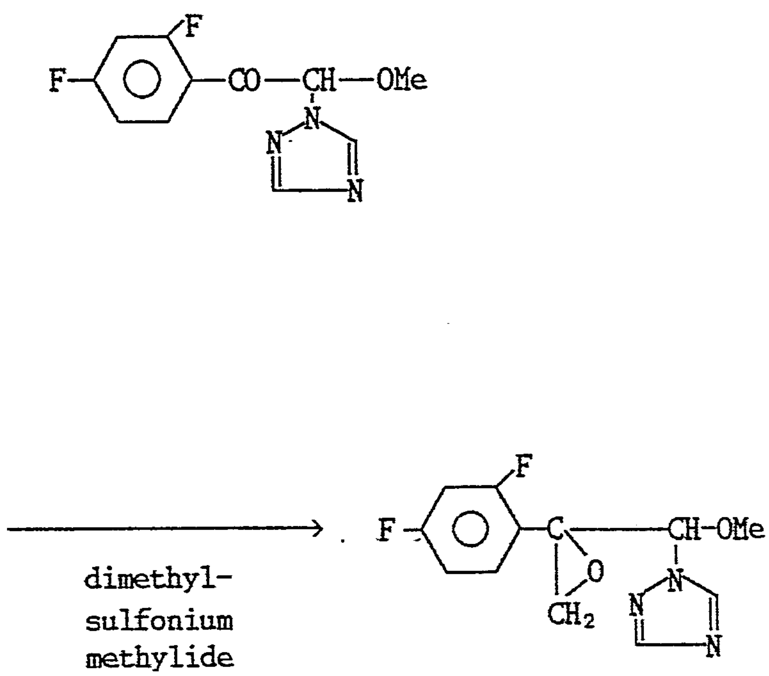

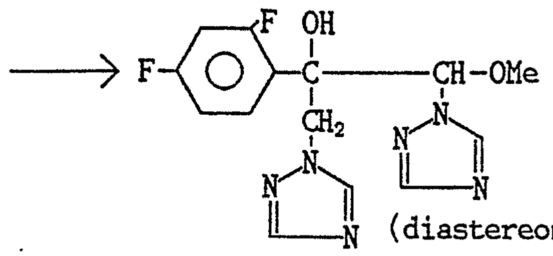

(diastereomer B)

To a solution of 1 g of 2',4'-difluoro-2-methoxy-2-(1H-1,2,4-triazol-1-yl)acetophenone in 10 ml of toluene are added 1.3 g of trimethylsulfoxonium iodide, 200 mg of benzyltriethylammonium chloride and 1.6 ml of 20 % aqueous sodium hydroxide and the mixture is stirred under heating at 60 °C for 1 hour. The reaction mixture is put into ice water and extracted with ether. The ether layer is washed with water, dried over sodium sulfate and concentrated to give 2-(2,4-difluorophenyl)-1-[1-methoxy-1-(1H-1,2,4-triazol-1-yl)methyl]oxirane.

To a suspension of sodium 1H-1,2,4-triazole made from 410 mg of 1H-1,2,4-triazole, 50 % sodium hydride (mineral oil suspension : 290 mg) and 4 ml of dry dimethylformamide is added the oxirane obtained above and the mixture is stirred at room temperature for 1.5 hr. The reaction mixture is put into ice water and extracted with methylene chloride. The organic layer is washed with water, dried over sodium sulfate and concentrated. The residue is subjected to silica gel column chromatography, eluting with 3 % methanol - methylene chloride and the eluate is concentrated. The residue is recrystallized from ethyl acetate - diisopropyl ether to give 426 mg of 2-(2,4-difluorophenyl)-1-methoxy-1,3-bis(1H-1,2,4-triazol-1-yl)-2-propanol (diastereomer B)

melting at 160.5 -164 °C as crystals.

Yield : 32 %

Anal Calcd. (%) for $C_{14}H_{14}N_6O_2F_2$ :

C, 50.00; H, 4.20; N, 24.99; F, 11.30

Found (%) : C, 49.89; H, 4.18; N, 24.73; F, 11.29

Examples 48 to 62

Diastereomer B, one of the two diastereomers of the objective compounds (I), is mainly obtained by the same reaction as in Example 47.

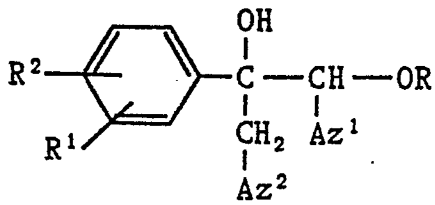

( I )

| Ex. No. | R | $R^1$ | $R^2$ | *1 $Az^1$ | *2 $Az^2$ | m.p. *9 (°C) |
|---|---|---|---|---|---|---|
| 48 | Me | H | 4-F | Tri | Tri | 112-122 *2 |
| 49 | Me | 2-F | 4-F | Py | Tri | 131-132 |
| 50 | Me | 2-F | 4-F | Py | Tri | 127-128 |
| 51 | Me | H | 2-F | Tri | Tri | 148.5-149.5 |
| 52 | Me | 2-Cl | 4-Cl | Tri | Tri | 158-180 *3 |
| 53 | Et | 2-Cl | 4-Cl | Tri | Tri | 190-191 |
| 54 | Et | 2-Cl | 4-Cl | Tri | Tri | 134-136 *4 |
| 55 | Me | 2-Cl | 4-Cl | Tri | Tri | 226-227 |
| 56 | Me | 2-F | 4-F | Im | Tri | 60-62 *5 |
| 57 | Me | H | 2-F | Tri | Tri | 157 *6 |
| 58 | Me | 2-Cl | 4-Cl | Im | Im | 204-206 *7 |
| 59 | $CH_2-CF_3$ | 2-F | 4-F | Tri | Tri | 174.5-176.5 |
| 60 | Me | H | $4-CF_3$ | Tri | Tri | 175-176 |
| 61 | Me | 2-F | 4-F | Tri | Tri | 160.5-164 |
| 62 | Me | H | 2-Cl | Tri | Tri | 151-187 *8 |

*1 The term "Tri" represents 1H-1,2,4-triazol-1-yl, "Im" represents 1H-imidazol-1-yl, and "Py" represents 1H-pyrazol-1-yl, respectively.

*2 Mixture of diastereomers A and B ( A/B = 0.5)

*3 Mixture of diastereomers A and B ( A/B = 1.0)

*4 Mixture of diastereomers A and B ( A/B = 2.0)

*5 Oxalate; 1 mol of oxalic acid, 1 mol of water and 1/2 mol of diethyl ether adduct; mixture of diastereomers A and B ( A/B = 0.5)

*6 Oxalate; 2 mol of oxalic acid and 2/5 mol of water adduct

*7 Mixture of diastereomers A and B ( A/B = 0.5)

*8 Mixture of diastereomers A and B ( A/B = 0.25)

*9 Pure diastereomer B unless otherewise mentioned.

Data of Activity

The compound bearing Compound Number used in the following Experiments is identified with the compound obtained in the corresponding Example No.

Experiment 1

Inhibition test against pseudomycelium formation of *Candida albicans*

*Candida albicans* KE-2 was inoculated to Eagle-MEM medium (Nissan 2 made by Nissui Seiyaku, Ltd.) supplemented with 20 % calf serum so that its final inoculum of yeast cell might be $1 \times 10^6$ yeast cells per milliliter. The test compounds are added thereon in two fold dilution series. After incubation for 18 hours at 37 °C, the fungal growth in each drug concentration is smeared and fixed on a slide glass, and its morphological feature on the presence or absence of the pseudomycelium formation is examined by a microscope following Giemsa staining. Minimum effective concentration (MEC, $\mu$g/ml) is defined as the lowest

concentration of the compound which prevented typical pseudo-mycelium formation.

Result

| Compd. No. | Inhibition for Pseudomyselium Formation of Candida albicans (MEC, μg/ml) |
|---|---|
| 21 | 0.63 |
| 23 | 0.63 |
| 24 | 1.25 |
| 25 | 0.31 |
| 28 | 0.08 |
| 29 | 0.31 |
| 30 | 0.31 |
| 31 | 1.25 |
| 33 | 1.25 |
| 34 | 0.63 |
| 35 | 0.04 |
| 36 | 0.08 |
| 37 | 0.63 |
| 38 | 0.31 |
| 46 | 0.08 |
| 47 | 0.31 |
| 49 | 0.31 |
| 51 | 0.63 |

Experiment 2

Therapeutic effect against experimental candidiasis in mice

After *Candida albicans* KE-2 is incubated on Sabouraud's glucose agar at 28 °C for 48 hr., it is suspended in Sabouraud's glucose broth and the cells ($2 \times 10^6$) are injected to a tail vein of Jcl-ICR female mice (4 weeks age ; weight : 18 - 20 g). A suspension of the test compound in 2 % gum arabic is orally administered three times, immediately after infection of the yeast ( 0 hour), after 4 hours and after 24 hours, as a dosage of 50 mg/kg. Non-treated group is used as control. Eight mice per group are used in the control group and the treated group. All the mice in the control group died within 48 hours after infection.

Therapeutic effect of the test compound is judged by mouse survival ratio on the 5th day after infection.

Result

| Compd. No. | Therapeutic Effect (%) |
|---|---|
| 21 | 87.5 |
| 24 | 87.5 |
| 25 | 100.0 |
| 26 | 100.0 |
| 27 | 25.0 |
| 28 | 62.5 |
| 29 | 100.0 |
| 30 | 50.0 |
| 31 | 37.5 |
| 32 | 50.0 |
| 35 | 25.0 |
| 36 | 37.5 |
| 37 | 62.5 |
| 38 | 25.0 |
| 40 | 37.5 |
| 44 | 12.5 |
| 45 | 37.5 |
| 46 | 12.5 |
| 49 | 75.0 |
| 54 | 87.5 |

As the result of the above embodiment of this invention, the objective compounds ( I ) are useful as human or veterinary medicinals, showing excellent antifungal activity.

CLAIMS

1. A compound of the general formula I:

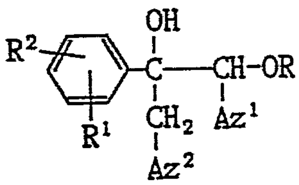

(I)

(wherein $Az^1$ and $Az^2$ each is imidazolyl, triazolyl or pyrazolyl, each optionally substituted by methyl or halogen;

R is $C_1$-$C_5$ alkyl or $C_1$-$C_5$ haloalkyl;

$R^1$ is hydrogen, halogen or trifluoromethyl and

$R^2$ is hydrogen or halogen)

or its salt.

2. A compound according to Claim 1, in which R is $C_1$-$C_5$ alkyl.

3. A compound according to Claim 2, in which $R^1$ is hydrogen.

4. A compound according to Claim 3, in which $R^2$ is halogen.

5. A compound according to Claim 4, namely 2-(4-chlorophenyl)-1-methoxy-1,3-bis(1H-1,2,4-triazol-1-yl)-2-propanol.

6. An antimycotic composition comprising a pharmacologically effective amount of a compound according to Claim 1 as an effective ingredient in association with one or more pharmaceutically acceptable carriers, diluents and / or excipients.

7. A compound of the general formula I according to any one of claims 1 to 5 for use in the treatment of mycosis.

8. A process for preparing a compound according to Claim 1 which comprises reacting a compound of the general formula IV:

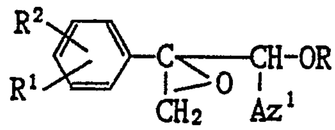 (IV)

(wherein R, $R^1$, $R^2$ and $Az^1$ each has the same meaning as defined above)
with an azole in the presence of a base in an appropriate solvent.